# EUROPEAN PATENT SPECIFICATION

(11) **EP 3 348 244 B1**
(45) Date of publication and mention of the grant of the patent: **06.08.2025**
(21) Application number: 18151830.9
(22) Date of filing: 16.01.2018
(51) Int. Cl.: A61F 9/02

(54) **GLASSES FOR SHIELDING AGAINST IONIZING RADIATION AND FRAME FOR USE IN A PAIR OF GLASSES**
SCHUTZBRILLE GEGEN IONISIERENDE STRAHLUNG UND BRILLENGESTELL ZUR BENUTZUNG IN EINER SCHUTZBRILLE
LUNETTES PROTECTRICES CONTRE LE RAYONNEMENT IONISANT ET MONTURE DE LUNETTES DESTINÉE À ÊTRE UTILISÉE DANS LES LUNETTES

(30) Priority: 16.01.2017 NL 2018172
(43) Date of publication of application: 18.07.2018
(73) Proprietor: MDT Medical Development & Technology Medical Instruments Division B.V., 5081 CT Hilvarenbeek (NL)
(72) Inventor: JANSSEN, Dion Paul Marie, 5081 CT Hilvarenbeek (NL)
(74) Representative: Patentwerk B.V.

(56) References cited:
- EP-A2- 2 261 727
- EP-A2- 2 775 193
- JP-A- 2003 004 892
- JP-A- 2012 225 702
- US-A- 4 021 862
- US-A- 4 024 405
- US-A- 5 548 125
- US-A1- 2004 004 196
- US-A1- 2004 147 652
- US-A1- 2011 261 313

## Description

The present invention relates to glasses for shielding a wearer thereof against ionizing radiation, in particular X-radiation, comprising a frame and at least one ionizing radiation shielding lens, such as a leaded glass lens, which lens is at least partially enclosed by the frame. The invention further relates to a frame for use in a pair of such glasses.

Ionizing electromagnetic radiation poses a great health hazard to those who must work around scanners, test and measurement instruments, fluoroscopes, X-ray machines and other radiation emitting equipment. Especially medical professionals such as cardiologists and radiologists are at an increased risk of contracting radiation associated diseases due to compounded radiation exposure as a result of long procedures and multiyear careers using radiation procedures. To effectively reduce exposure to the ionizing radiation, the radiation is commonly shielded by means of leaded aprons or suits and thyroid collars. As research has shown that said medical professionals have an increased risk of developing brain tumours and cataracts, there is a need for additional protection of the head and especially the eye region, as the eyes are one of the most sensitive areas of the body, and one of the most vulnerable to radiation.

US 4,021,862 for instance discloses a radiation eye shield adapted to protect the wearer from the hazards of direct beam and scattered radiation. US 2011/0261313 discloses a metal detectable lens carrier that includes a polymeric material and metal particulate dispersed throughout the polymeric material. US 2004/004196 describes articles, including fabrics and film layers, are disclosed which can protect against multiple hazards, including radiation, chemical, biological agents, metal projectiles and fire hazards. JP2012225702 describes a radiation protector for protecting a hippocampus of a user from radiation exposure. US 4,024,405 describes an X-ray eye shield useful in protecting eye tissue from radiation during dental radiography. US 2004/0147652 discloses a radiation material made of plastic material comprising a metallic filler.

For the purpose of protecting the eyes of medical professionals against the deleterious effects of radiation, radiation safety glasses are used that are commonly provided with leaded glass lenses that reduce the amount of radiation propagating through the lenses into the eyes of the wearer. Although these glasses are able to limit exposure of the eyes to incoming radiation, they are unable to limit this exposure up to a point where the involved health risks are sufficiently mitigated. In addition, current radiation safety glasses are often cumbersome in use and uncomfortable to wear, thereby hindering the medical professional in its work.

It is therefore a goal of this invention to provide glasses that offer an improved protection against ionizing radiation, and X-radiation in particular, that at the same time are comfortable to wear and do not hinder the wearer in any way.

This object can be achieved by providing glasses according to claim 1.

The use of a tungsten and plastic mixture as a material for the frame provides the frame with a high shielding effectiveness against incident ionizing radiation due to the high atomic number of tungsten. Tungsten may hereby serve as an alternative to lead, which has traditionally been used to shield healthcare practitioners.. Tungsten is nontoxic and nonreactive and can be effectively blended with plastics in high concentrations (up to 90% by weight) to produce a mixture or compound with a shielding effectiveness equal to or better than that of lead. A further advantage of tungsten is that it is reusable and, unlike lead, can be disposed of through normal channels. Nonetheless, it is conceivable that any metal, and in particular metals with a high atomic number including lead, may be used in the mixture, even instead of tungsten. The metal in the frame acts as absorbent of incoming radiation, thereby weakening, or even nullifying, this radiation and protecting the wearer of the frame. The higher the atomic number, the denser the metal, which increases the absorbing ability of the metal. Therefore metals with high atomic number, such as atomic number of 30 and above, are particularly effective in the present invention. The object of the invention can thus be achieved by providing glasses according to the preamble, wherein the frame is composed of a material comprising a mixture of at least a metal, in particular a heavy metal, and a plastic, preferably a plastic suitable to be injection moulded. Due to combining the metal with a plastic, a material (also referred to as "mixture" or "compound") is obtained that is highly formable and as such allows the glasses to be given complex shapes while retaining a consolidated design. The glasses can hence be designed to closely fit the shape of the wearer's head and be comfortable to wear for extended periods of time. In addition, the plastic provides the material with additional corrosion resistance and strength without unduly increasing the mass of the overall construction. The ability to reduce the weight of the glasses is especially important as the dense radiation shielding materials add weight to both the frame as well as the one or more lenses. For reasons of wearing comfort, it is however critical that the mass of the glasses is kept to a minimum. It is especially advantageous if the plastic applied is an injection mouldable plastic, as injection moulding allows the glasses to be given complex and intricate shapes that are otherwise too complicated and expensive to manufacture. Additionally, injection moulding is especially efficient due to the attainable high production output rates and the more or less finished appearance of the obtained product.

Polypropylene (PP) is favoured as plastic, in part by offering excellent mechanical properties in terms of strength and toughness, but also by offering superior flow and easy acceptance of high metal loadings. Additionally, polypropylene is able to withstand cleaning the frame with solutions containing at least up to 70% alcohol. It is however also possible to use a wide range of other plastics, including polyethylene (PE), polyamide (PA) and polyurethane (PU). In order to obtain a sufficient shielding effectiveness without unduly increasing the thickness of the material, tests have shown that the mixture or compound must at least contain 50 percent by weight metal, such as tungsten, and at most 50 percent by weight polypropylene. The shielding effectiveness of the material is hereby found to be sufficient when 95% of radiation emitted by a radiation source using a maximum voltage of 120 kV, placed at a distance of 1,5 metres, is absorbed or scattered. With metals other than tungsten, different percentage may apply based on the shielding effectiveness of the particular metals used. By further increasing the percentage by weight tungsten (or metal in general), the shielding effectiveness can either be increased or kept constant while decreasing the material thickness. The maximum percentage by weight of the metal typically lies in the range of 90-98% and is commonly determined by minimum percentage by weight of the plastic that is necessary for the flowability of the plastic to substantially remain intact such that the material remains mouldable.

Given the above-stated advantages of an injection moulding process, the frame is obtained by subsequently mixing and injection moulding of the tungsten and the plastic. In a commonly instance, the mixing comprises the plastic being loaded with powdered tungsten and/or another metal. The obtained mixture or compound has a high radiation shielding effectiveness due to the application of the dense metal, while still retaining a mouldability similar to that of the plastic. The subsequent moulding process allows the frame to be shaped in a single step and in high volume in a broad range of (complex) shapes. When possible due to the composition of the mixture or compound, the moulding process may be performed on standard injection moulding equipment.

In a possible embodiment of the glasses according to the invention, the plastic is polypropylene, and the mixture comprises at least 50 percent by weight (wt%) tungsten and at most 50 percent by weight (wt%) polypropylene. Polypropylene (PP) is favoured as plastic, in part by offering excellent mechanical properties in terms of strength and toughness, but also by offering superior flow and easy acceptance of high metal loadings. Additionally, polypropylene is able to withstand cleaning the frame with solutions containing at least up to 70% alcohol. It is however also possible to use a wide range of other plastics, including polyethylene (PE), polyamide (PA) and polyurethane (PU). In order to obtain a sufficient shielding effectiveness without unduly increasing the thickness of the material, tests have shown that the mixture or compound must at least contain 50 percent by weight tungsten and at most 50 percent by weight polypropylene. The shielding effectiveness of the material is hereby found to be sufficient when 95% of radiation emitted by a radiation source using a maximum voltage of 120 kV, placed at a distance of 1,5 metres, is absorbed or scattered. With metals other than tungsten, different percentage may apply based on the shielding effectiveness of the particular metals used. By further increasing the percentage by weight tungsten (or metal in general), the shielding effectiveness can either be increased or kept constant while decreasing the material thickness. The maximum percentage by weight of the metal typically lies in the range of 90-98% and is commonly determined by minimum percentage by weight of the plastic that is necessary for the flowability of the plastic to substantially remain intact such that the material remains mouldable.

In a further embodiment of the glasses according to the invention, the at least one ionizing radiation shielding lens is non-corrective. This commonly means that no difference exists in curvature between the front and rear surface of the radiation shielding lens. The fact that the one or more radiation shielding lenses of the glasses do not (need to) have a corrective function that necessitates cutting the lens to include a spherical correction according to the particular prescription of wearer significantly simplifies the manufacturing process for the glasses, thereby reducing manufacturing costs.

To enable the glasses to be used by wearers having eye conditions that necessitate the wearer to use prescription eyewear, the glasses may comprise an accessory frame placed in front of the at least one ionizing radiation shielding lens, which accessory frame is configured to enclose at least one corrective lens at least partially. Conventionally, the optical profile of the radiation shielding lens hereby differs from that of the corrective lens, wherein the radiation shielding lens preferably is non-corrective. By enabling the radiation shielding glasses to be used in concurrence with such an accessory frame, adaptation of the glasses to match the prescription of the wearer becomes possible and is significantly simplified in comparison with directly cutting the radiation shielding lens to include a spherical correction according to the particular prescription. Namely, as the refractive index of radiation shielding lenses, which in a common instance include lead, differs from that of plastic and glass lenses commonly used in prescription glasses, different cutting techniques and processes are necessary to produce these lenses. These techniques and processes are often not within the skills of ordinary opticians, meaning that changing the correction of the one or more lenses requires the skills of a specialist. With the prescription lenses being dissociated from the shielding lenses however, the corrective lenses could be easily adapted and replaced by any optician without having to alter the radiation shielding lenses.

The accessory frame may be placed on an inside of the frame turned towards the face of a wearer. An advantage of this relative position of the accessory frame is that the one or more corrective lenses held by the accessory frame can be placed at a distance from the eyes of the wearer conventional to normal prescription lenses. Moreover, as the accessory frame is place on an inside of the radiation shielding glasses, the exterior of the glasses can be kept clean without any protruding parts, which could facilitate the mounting of vision aids such as magnifying surgical loops.

In addition, the accessory frame may be detachably mounted on the frame to facilitate the removal and placement of the accessory frame from and on the radiation shielding glasses. The detachable mounting may be effectuated by means of mounting or attachment means provided on the accessory frame and/or the frame at least partially enclosing the one or more radiation shielding lenses. These mounting or attachment means may establish any connection between the respective frames such as a snap connection or a screw connection. It is however advantageous if the connection allows for quick and easy removal and placement of the accessory frame. It is also possible that the accessory frame is fixedly connected to the frame at least partially enclosing the one or more radiation shielding lenses, while the lenses held by the accessory frame are themselves detachably connected to said accessory frame. The accessory frame may be designed such that it can be used as prescription glasses independently from the radiation shielding glasses. It is even possible that the accessory frame is forms part of an ordinary pair of prescription glasses and that the radiation shielding glasses are provided with mounting means enabling the mounting of such prescription glasses to the radiation shielding glasses.

To be able to sufficiently limit radiation exposure up to a point where the involved health risks are mitigated, the frame may be configured to form an at least partially sealed interior around the eyes of a wearer. The glasses and the frame in particular may even form a substantially completely sealed interior around the eyes of the wearer. The interior around the eyes of a wearer and consequently the eyes of the wearer themselves are then protected against ionizing radiation entering the eyes not only from the front, but also from other directions possibly including the top, bottom and sides.

As a way to provide an adequate shielding around the eyes of the wearer of the glasses, the frame at opposing side ends may comprise temples for engaging either side of the wearer's head, wherein the temples comprise side panels dimensioned to form shields for shielding the eyes of a wearer from ionizing radiation entering from the side of the wearer's head. Said side panels preferably form an integrated part of the temples and therewith the frame, wherein the side panels are composed of the same material as the frame. It is possible that the side panels on their free edges connect to, and more preferably form-fit the head of the wearer to completely seal the outer sides of the eye sockets from incoming ionizing radiation.

The glasses may be configured to connect with a part of the face of a wearer, in particular below the eyes, preferably at the height of the cheekbones, to provide for additional shielding of the eyes from ionizing radiation, and especially radiation coming from below the wearer's head and propagating upwards. The connection between the glasses and the face may be achieved by providing the bottom end of the frame and/or the at least one lens with a shape that follows the shape of the face. Constituting a protruding part of the face, the cheekbones are a suitable area for the bottom end of the glasses to connect with, thereby preventing radiation to pass underneath the glasses and reach the eye area.

In a possible embodiment of the glasses according to the invention, the frame is provided with at least one flexible sealing element configured to connect on a first end with the face of a wearer, in particular below the eyes, and at a second end with a rim of the frame enclosing the ionizing radiation shielding lens. The sealing element may hereby be composed of a material comprising a similar mixture to that of the frame, possible added with a suitable elastomer or plasticizer to provide the sealing element with a degree of elasticity. The sealing element is flexible in order to closely adhere to the shape of the wearers face for increased protection. Moreover, the sealing element improves the fit of the glasses, which increases wearing comfort. Although radiation originating from below poses a predominant treat to medical professionals, as radiation emitting equipment conventionally targets part of a human body lying down wherein the radiation source is positioned at a lower level than the medical professional's eyes, similar flexible sealing elements may be applied on other parts of the glasses, and especially the edges of the frame where a gap is present between the glasses and the wearer's head.

The at least one sealing element may be provided with at least one reinforcing member, wherein the reinforcing member is deformable to at least partially form-fit the shape of the wearers face. The reinforcing member may for this purpose be made of a bendable material with good fatigue strength and plasticity. By deforming (bending) the one or more reinforcing members to attain a desirable shape for the sealing element, a customizable fit can be created without the need for customized glasses.

As the interior around the eyes of a wearer is sealed in an increased degree, fogging or steaming up of the lenses may occur due to exhaling or moisture and/or heat coming from the eyes and/or the skin. The at least one sealing element may therefore be provided with at least one ventilation channel connecting the inside to the outer side of the glasses to lead moist air away from the eye region and counteract this steaming or fogging up. The ventilation channel may hereby be configured to prevent propagation of ionizing radiation though the channel towards the inside of the glasses, wherein the channel in particular extends at an angle compared to direction of propagation of incoming ionizing radiation. As radiation propagates in a straight line, providing the channel with a large enough angle is an effective way in ensuring that the rays of radiation do not pass straight through the channel but instead pass through the sealing element, thereby being at least partly absorbed by said sealing element.

To further increase the fit and wearing comfort of the radiation shielding glasses, the glasses may comprise at least one nose pad, wherein the nose pad is moveably connected to the frame. The moveable connection between the frame and the nose pad allows the nose pad to move relative to the frame. This lets the nose pad adapt to the position of the nose, independent on the specific wearer. Moreover, the nose pad may accommodate for placement of the glasses in various positions (e.g. more forward or more backward placed) on the nose. The nose pad may additionally seal any existing gap between the glasses and the nose and/or other parts of the face. Moreover, the nose pad may be composed of a material with a high ionizing radiation shielding effectiveness.

In yet another embodiment of the glasses according to the invention, the glasses comprise a headband connected to the frame, wherein the headband is configured for fixing the glasses to the face of the wearer. The headband, preferably connected to the ends of either temple of the frame and placed around the back of the head allows for a secure fixation of the glasses to the wearer's face, even when hanging over a patient.

To ensure the medical professional with an unobstructed view looking through the glasses, the at least one ionizing radiation shielding lens may have a minimum light transmittance of 75%. This specified minimum light transmittance value is also preferred to meet various standards for eye protection, including the European norm EN166, which is considered the baseline for safety eyewear.

The invention further relates to a frame for use in a pair of glasses for shielding against ionizing radiation as described in any of the above-described embodiments, the advantages of which have been already discussed in the foregoing.

The invention will now be elucidated into more detail with reference to non-limitative exemplary embodiments shown in the following figures. In the figures:
- figure 1 shows a perspective view of a first embodiment of the glasses according to the invention as worn by a wearer, and
- figure 2a-2b respectively show a front and a rear perspective view of a second embodiment of the glasses according to the invention.

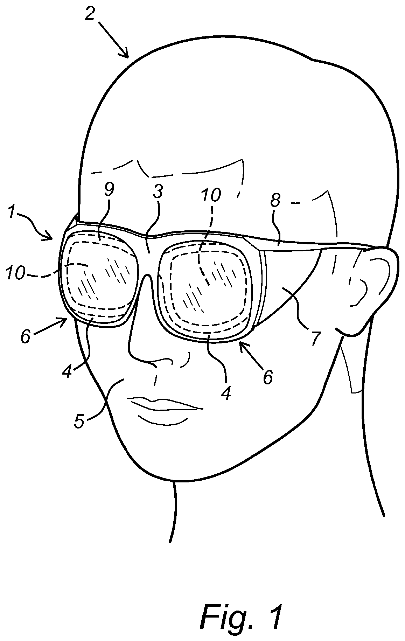

Figure 1 shows a perspective view of an embodiment of the glasses 1 for shielding against ionizing radiation according to the invention, wherein the glasses 1 are shown being worn by a wearer 2. The glasses 1 comprise a frame 3 that holds two ionizing radiation shielding lenses 4. It is however also possible that the frame 3 holds a different number of lenses, such as a single lens that covers both eyes. As a result of its particular shape, the frame 3 forms a sealed interior around the eyes of a wearer 2, in part by connecting with the face 5 of the wearer 2 in the cheekbone region 6, and in part by side panels 7 provided on the temples 8 of the frame 3. Due to the enclosure of the eyes created by the frame 3 and the lenses 4, the eyes are effectively shielded from ionizing radiation entering from all side of the wearer's head. Namely, the lenses 4 are typically made from a radiation shielding material such as leaded glass, while the frame 3 is composed of a material comprising a mixture of at least tungsten and a plastic, wherein especially the tungsten has a high radiation shielding effectiveness. An accessory frame 9 is placed on the inside of the frame 3 to hold two corrective lenses 10 that enable wearers with prescription eyewear to also use the radiation shielding glasses 1. The accessory frame 9 is optionally mounted to the frame 3 in a detachable manner.

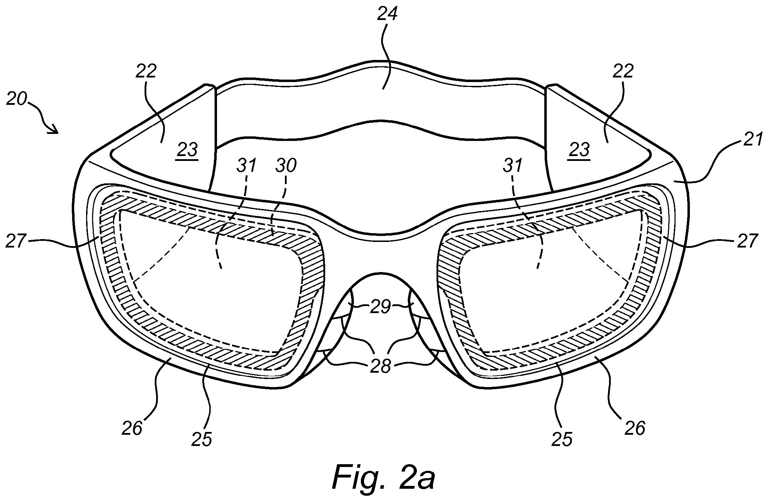

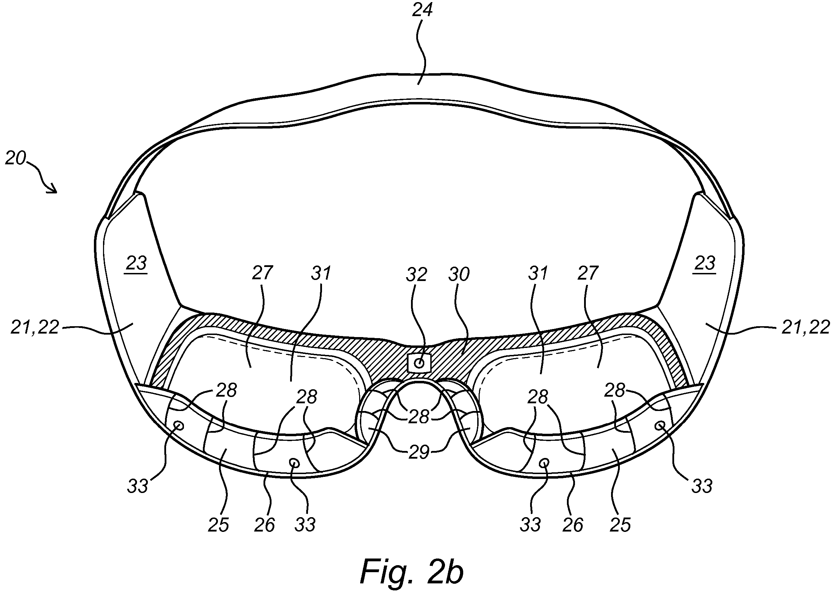

Figure 2a shows a front perspective view of another embodiment of the glasses 20 for shielding against ionizing radiation according to the invention. These glasses 20 again comprise a frame 21, composed of a radiation shielding material comprising a mixture of at least one metal and a plastic, wherein one of the at least one metal is preferably tungsten. The opposing side ends of the frame 21 comprise temples 22 that are configured to engage either side of the wearer's head. The temples 22 are widened to integrally form side panels 23 that shield the eyes of a wearer from ionizing radiation entering from the sides. At their back end, the temples 22 are mutually connected by means of a headband 24 that is configured to wrap around the head of a wearer and securely keeps the glasses 20 in place. At a lower end of the frame 21, flexible sealing elements 25 (clearly visible in figure 2b) are connected to the rims 26 of the frame 21 enclosing the ionizing radiation shielding lens 27. The sealing elements 25 are provided with reinforcing members 28 that may be deformed to follow the contours of the face, thereby realising a close connection between the wearer's face and the glasses 20. The glasses 20 also comprise nose pads 29, which nose pads 29 are preferable moveably connected to the frame 21 and may at their bottom ends connect to the sealing elements 25. In addition, the nose pads 29 may be made out of a similar flexible material as the sealing elements 25 and may be provided with deformable reinforcing members 28 to create a custom fit. The frame 21 holds two ionizing radiation shielding (leaded) lenses 27, which in this case are non-corrective. To facilitate using the glasses 20 despite having eye conditions that necessitate the use of prescription eyewear, an accessory frame 30 is provided on an inside of the glasses 20, which accessory frame 30 is configured to enclose one or more corrective lenses 31.

Figure 2b shows a rear perspective view of the embodiment of the glasses 20 shown in figure 2a. Elements corresponding to the ones shown and discussing in the figure 2a are herein indicated with corresponding numbers. Shown from behind, mounting means 32 are visible that are provided on the frame 21 and the accessory frame 30 configured to hold the corrective lenses 31. These mounting means 32 connect the accessory frame 30 to the outer frame 21 in a detachable manner. Although the mounting means 32 may establish any connection between the respective frames 21, 30, it is advantageous if the connection allows for quick and easy removal and placement of the accessory frame 30. The sealing elements 25 are furthermore provided with ventilation channels 33 connecting the inside to the outer side of the glasses 20 to prevent the lenses 27, 31 from steaming up.

It will be apparent that the invention is not limited to the exemplary embodiments shown and described here, but that within the scope of the appended claims numerous variants are possible which will be self-evident to the skilled person in this field. It is possible here to envisage that different inventive concepts and/or technical measures of the above described embodiment variants can be wholly or partially combined without departing from the inventive concept described in the appended claims.

## Claims

1. Glasses (1, 20) for shielding against ionizing radiation, in particular X-radiation, comprising:
- a frame (3, 21), and
- at least one ionizing radiation shielding lens (4, 27), such as a leaded glass lens, which lens (4, 27) is at least partially enclosed by the frame (3, 21),
wherein the frame (3, 21) is composed of a material comprising a mixture of at least tungsten and a plastic, **characterized in that** the plastic is polypropylene, polyethylene, polyamide or polyurethane, and the mixture comprises at least 50 wt% tungsten and at most 50 wt% plastic.

2. Glasses according to claim 1, wherein the frame (3, 21) is obtained by subsequently mixing and injection moulding of tungsten and the plastic.

3. Glasses (1, 20) according to any of the preceding claims, wherein the at least one ionizing radiation shielding lens (4, 27) is non-corrective.

4. Glasses (1, 20) according to any of the preceding claims, wherein the glasses (1, 20) comprise an accessory frame (9, 30) placed in front of the at least one ionizing radiation shielding lens (4, 27), which accessory frame (9, 30) is configured to enclose at least one corrective lens (4, 27).

5. Glasses (1, 20) according to claim 4, wherein the accessory frame (9, 30) is placed on an inside of the frame (3, 21) turned towards the face (5) of a wearer (2).

6. Glasses (1, 20) according to claim 4 or 5, wherein the accessory frame (9, 30) is detachably mounted on the frame (3, 21).

7. Glasses (1, 20) according to any of the preceding claims, wherein the frame (3, 21) is configured to form an at least partially sealed interior around the eyes of a wearer (2).

8. Glasses (1, 20) according to claim 7, wherein the frame (3, 21) at opposing side ends comprises temples (22) for engaging either side of the wearer's head, wherein the temples (22) comprise side panels (23) dimensioned to form shields for shielding the eyes of a wearer (2) from ionizing radiation entering from the side of the wearer's head.

9. Glasses (1, 20) according to claim 7 or 8, wherein the glasses (1, 20) are configured to connect with a part of the face (5) of a wearer (2), in particular below the eyes, preferably at the height of the cheekbones.

10. Glasses (1, 20) according to claim 9, wherein the frame (3, 21) is provided with at least one flexible sealing element (25) configured to connect on a first end with the face (5) of a wearer (2), in particular below the eyes, and at a second end with a rim of the frame (3, 21) enclosing the ionizing radiation shielding lens (4, 27).

11. Glasses (1, 20) according to claim 10, wherein the at least one sealing element (25) is provided with reinforcing members (28), wherein the reinforcing members (28) are deformable to at least partially form-fit the shape of the wearers face (5).

12. Glasses (1, 20) according to claim 10 or 11, wherein the at least one sealing element (25) is provided with at least one ventilation channel (33) connecting the inside to the outer side of the glasses (1, 20), wherein, preferably, the ventilation channel (33) is configured to prevent propagation of ionizing radiation though the channel (33) towards the inside of the glasses (1, 20), wherein the channel (33) in particular extends at an angle compared to direction of propagation of incoming ionizing radiation.

13. Glasses (1, 20) according to any of the preceding claims, wherein the at least one ionizing radiation shielding lens (4, 27) has a minimum light transmittance of 75%.

14. Frame (3, 21) for use in a pair of glasses (1, 20) for shielding against ionizing radiation according to any of the preceding claims, wherein the frame (3, 21) is composed of a material comprising a mixture of tungsten and a plastic, wherein the plastic is polypropylene, polyethylene, polyamide or polyurethane, and the mixture comprises at least 50 wt% tungsten and at most 50 wt% plastic and wherein the frame (3, 21) is obtained by subsequently mixing and injection moulding of the tungsten and the plastic.

## Patentansprüche

1. Brille (1, 20) zum Abschirmen gegen ionisierende Strahlung, insbesondere Röntgenstrahlung, umfassend:
- einen Rahmen (3, 21) und
- mindestens eine Abschirmlinse für ionisierende Strahlung (4, 27), wie beispielsweise eine Bleiglaslinse, wobei die Linse (4, 27) zumindest teilweise von dem Rahmen (3, 21) umschlossen ist, wobei der Rahmen (3, 21) aus einem Material besteht, umfassend ein Gemisch aus mindestens Wolfram und einem Kunststoff, **dadurch gekennzeichnet, dass** der Kunststoff Polypropylen, Polyethylen, Polyamid oder Polyurethan ist und das Gemisch mindestens 50 Gewichts-% Wolfram und höchstens 50 Gewichts-% Kunststoff umfasst.

2. Brille nach Anspruch 1, wobei der Rahmen (3, 21) durch anschließendes Mischen und Spritzgießen von Wolfram und dem Kunststoff erlangt wird.

3. Brille (1, 20) nach einem der vorstehenden Ansprüche, wobei die mindestens eine Abschirmlinse für ionisierende Strahlung (4, 27) nicht korrigierend ist.

4. Brille (1, 20) nach einem der vorstehenden Ansprüche, wobei die Brille (1, 20) einen Zubehörrahmen (9, 30) umfasst, der vor der mindestens einen Abschirmlinse für ionisierende Strahlung (4, 27) platziert ist, wobei der Zubehörrahmen (9, 30) konfiguriert ist, um mindestens eine Korrekturlinse (4, 27) zu umschließen.

5. Brille (1, 20) nach Anspruch 4, wobei der Zubehörrahmen (9, 30) auf einer Innenseite des Rahmens (3, 21) platziert ist, die dem Gesicht (5) eines Trägers (2) zugewandt ist.

6. Brille (1, 20) nach Anspruch 4 oder 5, wobei der Zubehörrahmen (9, 30) lösbar an dem Rahmen (3, 21) montiert ist.

7. Brille (1, 20) nach einem der vorstehenden Ansprüche, wobei der Rahmen (3, 21) konfiguriert ist, um einen zumindest teilweise abgedichteten Innenraum um die Augen eines Trägers (2) zu bilden.

8. Brille (1, 20) nach Anspruch 7, wobei der Rahmen (3, 21) an gegenüberliegenden Seitenenden Bügel (22) zum Eingreifen mit beiden Seiten des Kopfs des Trägers umfasst, wobei die Bügel (22) Seitenplatten (23) umfassen, die dimensioniert sind, um Abschirmungen bilden, um die Augen eines Trägers (2) vor ionisierender Strahlung abzuschirmen, die von der Seite des Kopfs des Trägers eintritt.

9. Brille (1, 20) nach Anspruch 7 oder 8, wobei die Brille (1, 20) konfiguriert ist, sich mit einem Teil des Gesichts (5) eines Trägers (2), insbesondere unterhalb der Augen, vorzugsweise auf Höhe der Wangenknochen, verbinden zu sein.

10. Brille (1, 20) nach Anspruch 9, wobei der Rahmen (3, 21) mit mindestens einem flexiblen Dichtungselement (25) versehen ist, das konfiguriert ist, um an einem ersten Ende mit dem Gesicht (5) eines Trägers (2), insbesondere unterhalb der Augen, und an einem zweiten Ende mit einem Rand des Rahmens (3, 21), der die Abschirmlinse für ionisierende Strahlung (4, 27) umschließt, verbunden zu sein.

11. Brille (1, 20) nach Anspruch 10, wobei das zumindest eine Dichtungselement (25) mit Verstärkungselementen (28) versehen ist, wobei die Verstärkungselemente (28) verformbar sind, um zumindest teilweise formschlüssig mit der Form des Gesichts (5) des Trägers zu sein.

12. Brille (1, 20) nach Anspruch 10 oder 11, wobei das zumindest eine Dichtungselement (25) mit zumindest einem Belüftungskanal (33) versehen ist, der die Innenseite mit der Außenseite der Brille (1, 20) verbindet, wobei vorzugsweise der Belüftungskanal (33) konfiguriert ist, um eine Ausbreitung von ionisierender Strahlung durch den Kanal (33) zu der Innenseite der Brille (1, 20) zu verhindern, wobei sich der Kanal (33) insbesondere in einem Winkel im Vergleich zu einer Ausbreitungsrichtung einfallender ionisierender Strahlung erstreckt.

13. Brille (1, 20) nach einem der vorstehenden Ansprüche, wobei die mindestens eine Abschirmlinse für ionisierende Strahlung (4, 27) eine minimale Lichtdurchlässigkeit von 75 % aufweist.

14. Rahmen (3, 21) zur Verwendung in einer Brille (1, 20) zur Abschirmung gegen ionisierende Strahlung nach einem der vorstehenden Ansprüche, wobei der Rahmen (3, 21) aus einem Material besteht, umfassend ein Gemisch aus Wolfram und einem Kunststoff, wobei der Kunststoff Polypropylen, Polyethylen, Polyamid oder Polyurethan ist und das Gemisch mindestens 50 Gewichts-% Wolfram und höchstens 50 Gewichts-% Kunststoff umfasst und wobei der Rahmen (3, 21) durch anschließendes Mischen und Spritzgießen des Wolframs und des Kunststoffs erlangt wird.

## Revendications

1. Lunettes (1, 20) de protection contre les rayonnements ionisants, en particulier les rayonnements X, comprenant :
- une monture (3, 21), et
- au moins un verre (4, 27) de protection contre les rayonnements ionisants, comme un verre en verre au plomb, lequel verre (4, 27) est au moins partiellement enfermé par la monture (3, 21), dans lesquelles la monture (3, 21) est composée d'un matériau comprenant un mélange d'au moins du tungstène et un plastique, **caractérisées en ce que** le plastique est du polypropylène, du polyéthylène, du polyamide ou du polyuréthane, et le mélange comprend au moins 50 % en poids de tungstène et au plus 50 % en poids de plastique.

2. Lunettes selon la revendication 1, dans lesquelles la monture (3, 21) est obtenue par mélange et moulage par injection ultérieurs de tungstène et du plastique.

3. Lunettes (1, 20) selon l'une quelconque des revendications précédentes, dans lesquelles l'au moins un verre (4, 27) de protection contre les rayonnements ionisants est non correcteur.

4. Lunettes (1, 20) selon l'une quelconque des revendications précédentes, dans lesquelles les lunettes (1, 20) comprennent une monture accessoire (9, 30) placée devant l'au moins un verre (4, 27) de protection contre les rayonnements ionisants, laquelle monture accessoire (9, 30) est configurée pour enfermer au moins un verre (4, 27) correcteur.

5. Lunettes (1, 20) selon la revendication 4, dans lesquelles la monture accessoire (9, 30) est placée sur un intérieur de la monture (3, 21) tournée vers le visage (5) d'un porteur (2).

6. Lunettes (1, 20) selon la revendication 4 ou 5, dans lesquelles la monture accessoire (9, 30) est montée de manière amovible sur la monture (3, 21).

7. Lunettes (1, 20) selon l'une quelconque des revendications précédentes, dans lesquelles la monture (3, 21) est configurée pour former un intérieur au moins partiellement scellé autour des yeux d'un porteur (2).

8. Lunettes (1, 20) selon la revendication 7, dans lesquelles la monture (3, 21) aux extrémités latérales opposées comprend des branches (22) pour venir en prise de chaque côté de la tête du porteur, dans lesquelles les branches (22) comprennent des panneaux latéraux (23) dimensionnés pour former une protection pour protéger les yeux d'un porteur (2) des rayonnements ionisants entrant du côté de la tête du porteur.

9. Lunettes (1, 20) selon la revendication 7 ou 8, dans lesquelles les lunettes (1, 20) sont configurées pour entrer en contact avec une partie du visage (5) d'un porteur (2), en particulier sous les yeux, de préférence à la hauteur des pommettes.

10. Lunettes (1, 20) selon la revendication 9, dans lesquelles la monture (3, 21) est pourvue d'au moins un élément d'étanchéité(25) flexible configuré pour venir en contact à une première extrémité avec le visage (5) d'un porteur (2), en particulier sous les yeux, et à une seconde extrémité avec un rebord de la monture (3, 21) enfermant le verre (4, 27) de protection contre les rayonnements ionisants.

11. Lunettes (1, 20) selon la revendication 10, dans lesquelles l'au moins un élément d'étanchéité (25) est pourvu d'éléments de renforcement (28), dans lesquelles les éléments de renforcement (28) sont déformables pour s'adapter au moins partiellement à la forme du visage (5) du porteur.

12. Lunettes (1, 20) selon la revendication 10 ou 11, dans lesquelles l'au moins un élément d'étanchéité (25) est pourvu d'au moins un canal (33) de ventilation reliant l'intérieur au côté externe des lunettes (1, 20), dans lesquelles, de préférence, le canal (33) de ventilation est configuré pour empêcher la propagation des rayonnements ionisants à travers le canal (33) vers l'intérieur des lunettes (1, 20), dans lesquelles le canal (33) s'étend en particulier selon un angle par rapport à la direction de propagation des rayonnements ionisants entrants.

13. Lunettes (1, 20) selon l'une quelconque des revendications précédentes, dans lesquelles l'au moins un verre (4, 27) de protection contre les rayonnements ionisants présente une transmittance lumineuse minimale de 75 %.

14. Monture (3, 21) destinée à être utilisée dans une paire de lunettes (1, 20) de protection contre les rayonnements ionisants selon l'une quelconque des revendications précédentes, dans laquelle la monture (3, 21) est composée d'un matériau comprenant un mélange de tungstène et d'un plastique, dans laquelle le plastique est du polypropylène, du polyéthylène, du polyamide ou du polyuréthane, et le mélange comprend au moins 50 % en poids de tungstène et au plus 50 % en poids de plastique et la monture (3, 21) étant obtenue par mélange et moulage par injection ultérieurs du tungstène et du plastique.
